(19) Europäisches Patentamt
European Patent Office
Office européen des brevets

(11) **EP 4 491 100 A1**

(12) **EUROPEAN PATENT APPLICATION**

(43) Date of publication:
**15.01.2025 Bulletin 2025/03**

(21) Application number: **23382718.7**

(22) Date of filing: **13.07.2023**

(51) International Patent Classification (IPC):
**A61B 3/11** *(2006.01)* **A61B 3/113** *(2006.01)*
**A61B 3/14** *(2006.01)* **G02B 27/01** *(2006.01)*

(52) Cooperative Patent Classification (CPC):
**A61B 3/113; A61B 3/112; A61B 3/14;**
**G02B 27/017;** G02B 2027/0178

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Nura Technology, S.L.**
**08005 Barcelona (ES)**

(72) Inventor: **ANTOLÍ GONZÁLEZ, Nuria**
**08005 Barcelona (ES)**

(74) Representative: **Herrero & Asociados, S.L.**
**Edificio Aqua - Agustín de Foxá, 4-10**
**28036 Madrid (ES)**

(54) **OPTICAL DEVICE FOR MONITORING EYE HEALTH**

(57) The optical device for monitoring eye health includes a frame (1) in which ophthalmic lenses (2) coated with an infrared filter are mounted; infrared light emitters (7) that illuminate an inner face of the lenses (2), reflecting the light towards the eyes to create images by reflecting off the inner face of the lenses (2); cameras (3) that focus the images created by reflecting off the inner face of the lenses (2); and sensors (5) that detect, as data, the images recorded by the cameras (3); cameras (3) that focus the images created by the reflection of the inner face of the lenses (2); and sensors (5) that detect as data, such images of the eyes recorded by the cameras (3), sending the detected data to an external electronic device (9).

It allows to determine from eye movement and other parameters, the eye health of a user's eyes to make an early detection of visual anomalies and other pathologies.

FIG. 1

## Description

## Object of the invention

The present invention relates to an optical device for monitoring eye health, being able to determine from eye movement and other parameters, the eye health of a user to make an early detection of visual anomalies and other pathologies and making use of artificial intelligence to facilitate prevention by a medical team and alert a patient/user through direct connectivity and communication in real time.

## Background to the invention

In order to diagnose the eye health of a person, it is necessary to carry out an examination in an ophthalmologist's surgery using specially designed equipment.

In the surgery, the ophthalmologist can determine if there is an eye health problem based on the information obtained in a short period of time, which is the time the patient spends in the surgery, but it does not allow for continuous monitoring of the patient's eye health.

Similarly, when the optometrist performs the vision test in his or her optician's surgery with his or her instrumentation, he or she can determine if there is an eye health problem during the time the patient is in the surgery, but it does not allow for continuous monitoring of the patient's eye health.

On the other hand, the implementation of so-called "smart glasses" is being studied, which incorporate technology that allows them to integrate functions very similar to those offered by a smartphone.

Another of the functionalities that can be found in existing smart glasses on the market is the ability to capture images and record videos, even offering the possibility of sharing them on social networks directly from the glasses themselves.

In addition, smart glasses on the market can also make and receive calls, facilitating communication.

That is, there are already smart glasses on the market today that are designed to provide an enhanced user experience, but they are not designed to provide information about the users' eye health.

## Description of the invention

Therefore, the aim of the present invention is to provide a wearable optical device for monitoring eye health as a substitute for prescription glasses as we know them today. A user will wear the optical device as a regular smartwatch, which can be activated for an extended period of time to capture data (images) of their eyes, allowing real-time information to be sent to an ophthalmologist or optometrist to determine a possible ocular pathology or to follow up on their visual health or a post-operative procedure.

With this optical device, the user's eyes can be monitored according to parameters set by optometrists or ophthalmologists, which will lead to improvements in habits or health through connectivity between the doctor and the patient, contributing to the well-being and health of the wearer of the eye monitoring device.

With the optical device of the invention, the above-mentioned disadvantages are solved and other advantages are described below.

The optical device in accordance with the present invention is defined in claim 1, and the dependent claims include additional features that are optional.

In particular, the eye health monitoring device comprises:

- a frame with integrated electronics in which prescription ophthalmic lenses are mounted. Conveniently, the ophthalmic lenses used must be coated with an IR infrared filter, narrow optical filters with respective illumination;
- IR light emitters (diodes) that illuminate the inner side of the lenses, reflecting the light towards the eyes to obtain a quality level of visualization of the eye through a reflection of the inner side of an optometric lens;

- cameras that focus the images created by the reflection of the inner side of the lenses and to improve the contrast of the image of the eye with respect to the ambient light, an active illumination system is implemented. This requires an LED, preferably in IR, and in a compact, low-power, miniature, SMD-type LEDs; and
- sensors that detect, as data, these images of the eyes recorded by the cameras, sending the detected data to an external electronic device, a computer or a smartphone.

According to the preferred embodiment, the frame comprises a Front End and Rods, in which is integrated a flexible electrical circuit with all the necessary components for eye tracking including cameras projecting light with an index of refraction "x", nanosensors and battery (power supply) that powers the cameras, light emitters and sensors.

Preferably, the emitters of the light emitters (LED lighting system) emit in infrared wavelength, between 800 - 820 nm, to avoid interfering with wavelengths detected by the human eye, ranging from 350 to 780 nm. The filter coating on the inner side of the lenses provides a reflection of infrared radiation greater than 90 % and a visible transmission greater than 90 %.

According to the present invention, with the optical device the user's eyes can be monitored, obtaining and processing data to be sent to optometrists and ophthalmologists. This data processing automation process refers to the design of algorithms that will contrast the image data received with parameterized clinical images of anonymous visual dysfunctions and pathologies to facilitate prevention by the medical team and alert the patient/user through direct connectivity and communication in real time.

The captured data is analyzed by software that allows alert levels to be set so that the medical team can detect and analyze data to prevent the growth of ocular dysfunction and alert their patient in real time.

Visual dysfunctions subject to study and monitoring may include: Myopia (stabilization), dry eye (prior to myopia), early cataracts, retinal detachment, glaucoma, diabetic retinopathy, macular degeneration, among others indicated in the future by ophthalmology medical teams or optometrists.

**Brief description of the drawings**

For a better understanding of what has been explained above, some drawings are included in which, schematically and only by way of a non-limiting example, a practical case of embodiment is represented.

Figure 1 is a perspective view of the monitoring device according to the present invention; and

Figure 2 is an optical schematic of the monitoring device according to the present invention.

**Description of preferred application**

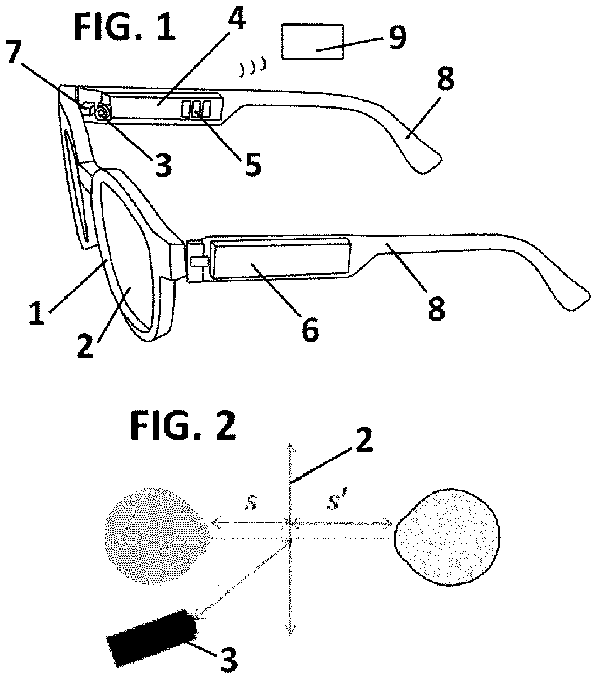

Figure 1 shows the optical device for monitoring eye health according to the present invention, in particular, glasses comprising a frame 1 and ophthalmic lenses 2.

Mounted on the mount 1 are cameras 3, an electronic circuit 4, sensors 5, a battery 6 (which powers all the components of the device) and light emitters 7.

The cameras 3, which are at least two, one for each eye, are located on the inner side of temples 8 of the frame (figure 1 of the glasses). The rest of the components are also preferably located on the temples 8 of the frame 1.

Each camera 3 preferably has a variable zoom, i.e., the focusing distance of the camera will vary according to the radius of curvature subject to the graduation of the lens, with a minimum frequency of e.g., 30 fps (frames per second).

The camera 3 preferably comprises a zoom lens (variable focal length), for example, between 25 and 740 mm, to focus the eye whether the person is nearsighted, farsighted or farsighted. The variable focal length calculations of the lens can be in the range of myopic to -10D and farsighted to +7D, using the base radii of the most commonly used ametropia-correcting lenses.

The study of the optical device shall start with a narrow range that can be addressed with a fixed focal length optic and a certain limited focusing range. One criterion for determining the range is to start by focusing on the most common refractive error of the initial target customer segment.

In the longer term, different optical solutions for different refractive error ranges will be realized. This will also help to contain the dimensions of the camera.

In turn, each light emitter 7 may be a light emitting diode (LED) emitting infrared radiation with a wavelength of, for example, 800-820nm. Each light emitter 7 may be integrated in the camera 3, integrated in the temples 8 of the mount 1 or may be integrated in any part of the frame 1, also in the front part of the mount.

Lenses 2 must have a coating or filter on their inner side, with a reflection of infrared radiation of e.g., 90 % higher and a visible transmission of e.g., 90 % higher.

The sensors 5 are preferably mounted on the electronic circuit 4 which are wirelessly connected to an external electronic device (not shown in the drawings), e.g., a computer, a mobile phone, a tablet, etc.

The operation of the monitoring device according to the present invention is as follows.

First, the light emitters 7 illuminate the inner side of the lens 2 which is coated with a filter that reflects infrared radiation and acts as a mirror for that wavelength, reflecting the light towards the eye.

Lens 2 creates the image of the eye at a certain distance, which will be where camera 3 will be focused, as shown in figure 2, being:

$$1/s + 1/s' = 1/f$$

$$F = R/2$$

Where according to figure 2:

s = distance from the eye to the lens;
s' = distance from the eye to the lens;
f = focal length of the lens; and
R = radius of curvature of the lens.

In a particular case where the lens is flat, then R = ∞ and f = ∞, where s = -s'.

The image is formed at the same distance as the eye.

In a standard case, the distance from the vertex of the eye to the lens is equal to, for example, s = 14 mm. Therefore, the camera will be in focus at a distance equal to the distance between the camera and the lens, i.e., 14 mm.

The following table shows an example of the zoom range of the camera:

|  | Minimum | Maximum |
|---|---|---|
| Radius (mm) | 50 | 1480 |
| Focal (mm) | 25 | 740 |

(continued)

|  | Minimum | Maximum |
|---|---|---|
| s' (mm) | -32 | -14 |

For a hyperopic range of +7D to a myopic range of -10D, the focusing range of the camera is between 14 and 32 mm. Considering that the size of the human eye is approximately 25 mm in diameter (y), we obtain the size of the image of the eye through the lens (y').

$$y'/y = - s'/s$$

|  | Minimum | Maximum |
|---|---|---|
| Radius (mm) | 50 | 1480 |
| Focal (mm) | 25 | 740 |

| s' (mm) | -31,8 | -14,3 |
|---|---|---|
| y' | 56,8 | 25,5 |

The images captured with the technological optical device are sent to the external electronic device, where they can be consulted by an ophthalmologist or optometrist, who will be able to determine whether or not there is an ocular dysfunction. This data will be previously analyzed and interpreted by means of an artificial intelligence system that will contrast the images obtained from the monitoring of the patient's eyes with parameterized clinical images to facilitate the conclusions and decisions of the ophthalmologist or optometrist. In other words, the device will facilitate the early detection of visual anomalies, favoring personalized preventive medicine and quality care between the healthcare team and the patient/user.

The following are non-exhaustive examples of how data are recorded and evaluated:

- Eye movement:
  The device uses eye tracking to determine the direction and speed of eye movement. For example, if a user looks to the right, the system records eye movement in that direction. If the user makes rapid, repetitive eye movements, the system also records these movements. This can be useful for detecting eye disturbances such as nystagmus, an involuntary, repetitive eye movement or a flare-up of ADHD (Attention Deficit Disorder).

- Pupillary diameter:
  The camera captures images of the eyes, and the diameter of the pupils is analyzed. Pupil diameter can vary in response to different lighting conditions and emotions. The system monitors these changes to detect possible abnormalities, such as excessive pupil constriction or dilation that could indicate an eye or neurological condition. In addition, algorithms and detection models compare eye movement and pupil diameter data to pre-defined normal ranges. If any significant deviation from normal ranges is detected, an alert is issued indicating a possible visual disturbance and recommending further medical evaluation.

- Measuring time in front of a screen:
  To measure the time a person spends in front of a screen and compare it with the hours spent in outdoor activities, a time recording system and an analysis algorithm can be used that responds to a flicker frequency within normal parameters.

The device may comprise an integrated ambient light sensor and timer. The ambient light sensor measures the intensity of light reaching the device, and the timer records the duration of time the device is on.

The device uses the ambient light sensor to identify when the user is in front of a screen. For example, when the light intensity exceeds a certain threshold, the user is considered to be using a screen. This can include computer screens, tablets, smartphones or other electronic devices.

The analysis algorithm records the total time the user spends in front of a screen and compares it to the time spent in outdoor activities. The algorithm can use predefined usage patterns to determine the recommended time for each type of activity. For example, a maximum of 2 hours a day in front of a screen would be within the acceptable healthy usage time, while a minimum of 1 hour a day is recommended for outdoor activities.

If the total screen time exceeds the threshold set as healthy, the device can issue a visual or audible alert to inform the user about excessive screen time. In addition, the device can provide recommendations on the importance of balancing screen time with outdoor activities to maintain a healthy lifestyle and to introduce a healthy habit warning to avoid Visual Fatigue the 20/20/20 Method (every 20 minutes spent looking at a screen, look at something 6 meters (20 feet) away for 20 seconds). (At this point it would be interesting for the eye care professional (ophthalmologist/optometrist) to indicate what percentage of visual capacity is lost with bad habits or to establish a relationship between hours in front of a screen equal to a percentage of vision loss).

- Myopia in children:

  To address the accelerated growth of myopia in children due to unhealthy habits, such as spending more than 8 hours a day in front of screens and not exercising, we can consider implementing the following measures in the device:

The device can include a reminder system that alerts children when they have exceeded a set time limit in front of screens, and the time limit can be customised according to the recommendations of eye health experts for each age group.

The device may have a special eye protection mode that automatically adjusts the brightness, contrast and blue light emitted by the screen. This helps reduce eyestrain and exposure to harmful blue light that has been linked to accelerated myopia growth in some studies.

The device can include physical activity sensors that record the level of movement and exercise the child is doing. It can track the amount of time spent in outdoor activities, such as playing, running or playing sports. This allows for a quantitative record of the amount of time children spend exercising.

Based on data collected about the user's screen viewing time and physical activity, the device has the ability to offer personalized recommendations. These suggestions could range from periodic reminders to take breaks and participate in outdoor activities to advice on age-appropriate games and physical activities.

In addition, the device could have a section dedicated to education on the importance of maintaining healthy habits for visual health, such as limiting screen time and participating in outdoor activities. In this section, valuable information and advice would be provided for both children and their parents, with the aim of maintaining a proper balance for their visual health.

Specific examples in terms of hours spent on screens and physical exercise:

Example 1:

- Screen time: 8 hours per day.
- Physical exercise: None.

Device action: The device will issue visual or audible alerts to remind the user that they have exceeded the recommended screen time and have not engaged in any physical activity. It can also suggest the importance of balancing screen time with outdoor activities and physical exercise to maintain optimal visual health and create a warning alert to the ophthalmologist/ optometrist resulting in a message from the doctor to the patient. This alert can be automated.
Example 2:

- Screen time: 8 hours per day.
- Physical exercise: Average of 3 hours per week.

Device action: The device can record screen time and physical exercise. If the user exceeds the recommended 8 hours of screen time per day or does not reach 3 hours of physical exercise per week, the device will issue alerts to remind the user of the importance of maintaining a proper balance between screen time and physical activity, and the user will also receive an alert from their ophthalmologist/ optometrist to follow up with their patient.
Example 3:

- Screen time: 3 hours per day.
- Physical exercise: 1 to 2 hours per day.
  Device action: The device monitors screen time and physical exercise. If the user exceeds the recommended 3 hours of screen time per day or fails to achieve 1-2 hours of physical exercise per day, an alert will be generated both on the device and in the ophthalmologist's or optometrist's records. This will allow for closer monitoring and personalized intervention to address the risk of accelerated myopia growth.
- Food:
  To include warnings and monitoring of healthy eating and its impact on eye health, the following measures can be considered.

Power logging: The device can have a function to record and track the user's food intake. This can be done through a connected app or platform that allows the user to enter and record the food consumed throughout the day.

The device can provide nutritional information and specific recommendations related to eye health. This may include advice on foods rich in vitamins and nutrients essential for eye health, such as vitamin A, vitamin C, vitamin E, omega-3, lutein, zeaxanthin, zinc, among others.

The device can analyze recorded dietary data and compare it to nutritional recommendations for a healthy, balanced diet. Using algorithms and models, the device can assess whether the user is getting the nutrients needed for eye health.

If it is detected that the user's diet does not meet the nutritional requirements for optimal eye health, the device can issue visual or audible alerts. These alerts can be weekly to provide regular monitoring and reminders to improve nutrition.

The device can provide personalized suggestions and recommendations for healthy foods that are beneficial to eye health. This can include recipes, shopping lists and tips for incorporating these foods into the daily diet.

It is important to note that dietary monitoring and alerts can be complemented by follow-up and recommendations from healthcare professionals, such as nutritionists or eye doctors. The device can collaborate with the medical team to provide relevant data and alerts for comprehensive and personalized care, coordinated by professionals carrying out vision therapy with their patients.

- Healthy eating detectable through the eyes:
 With the device we will be able to monitor and analyze the eyes to detect possible signs of nutrient deficiencies and dietary deficiencies that could affect eye health, considering the following:
 The device may use its ability to capture images of the eyes to perform detailed analysis (eye monitoring). This may include assessment of color, brightness, appearance of blood vessels, and other visual indicators related to eye health as indicated by medical equipment.

The artificial intelligence of the device can compare the results obtained from the user's eyes with predefined healthy parameters. These standards can be developed based on medical research and knowledge related to the effects of diet on eye health. Using image analysis algorithms, the device can identify potential signs of nutritional deficiencies, such as changes in eye color or appearance associated with a lack of key vitamins and nutrients.

If signs of nutritional deficiencies are detected in the user's eyes, the device can generate visual or audible alerts to inform the user about the importance of a healthy and balanced diet to maintain good eye health, and at the same time create alerts that the doctor will receive and communicate with their patient in a personalized way promoting personalized quality care. This can include suggestions for foods rich in vitamins and essential nutrients, as well as recipes or dietary guidelines.

Although reference has been made to a specific embodiment of the invention, it is obvious to a person skilled in the art that the monitoring device described is susceptible to numerous variations and modifications, and that all the details mentioned can be replaced by technically equivalent ones, without departing from the scope of protection defined by the appended claims.

## Claims

1. An optical device for monitoring eye health, comprising a frame (1) in which ophthalmic lenses (2) coated with an infrared filter are mounted, **characterized in that** the device also comprises:

   - infrared light emitters (7) that illuminate an inner face of the lenses (2), reflecting the light towards the eyes to create images by reflecting off the inner face of the lenses (2);
   - cameras (3) which focus the images created by the reflection of the inner face of the lenses (2);
   - sensors (5) that detect, as data, these images of the eyes recorded by the cameras (3), sending the detected data to an external electronic device (9).

2. Optical device for monitoring eye health according to claim, wherein the frame (1) comprises temples (8), on which the light emitters (7) and cameras (3) are mounted.

3. Optical device for monitoring eye health according to any one of the preceding claims, wherein the device also comprises an electronic circuit (4) in which the sensors (5) are mounted.

4. Optical device for monitoring eye health according to any one of the preceding claims, wherein the device also comprises a battery (6) that powers the cameras (3), light emitters (7) and sensors (5).

5. Optical device for monitoring eye health according to any one of the preceding claims, wherein the light emitters (7) are light emitting diodes, LEDs.

6. Optical device for monitoring eye health according to any one of the preceding claims, wherein the external electronic device (9) is a computer or a smartphone.

7. Optical device for monitoring eye health according to any one of the preceding claims, wherein the light emitters (7) emit at an infrared wavelength between 800 - 820 nm.

8. Optical device for monitoring eye health according to claim 1, wherein the coating of the lenses (2) provides an infrared radiation reflection greater than 90 % and a visible transmission greater than 90 %.

# FIG. 1

# FIG. 2

Europäisches
Patentamt

European
Patent Office

Office européen
des brevets

# EUROPEAN SEARCH REPORT

Application Number

EP 23 38 2718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 203 520 305 U (USTC UNIV SCIENCE TECH CN) 2 April 2014 (2014-04-02)<br>* paragraph [0019] – paragraph [0030] *<br>* figure 1 *<br>----- | 1-8 | INV.<br>A61B3/11<br>A61B3/113<br>A61B3/14<br>G02B27/01 |
| X | US 2017/332901 A1 (HWANG INSEOK [US] ET AL) 23 November 2017 (2017-11-23)<br>* paragraph [0012] – paragraph [0014] *<br>* paragraph [0016] – paragraph [0022] *<br>* paragraph [0027]; figures 1A-1C, 2 *<br>----- | 1-8 | |

TECHNICAL FIELDS
SEARCHED      (IPC)

A61B
G02B

The present search report has been drawn up for all claims

| Place of search | Date of completion of the search | Examiner |
|---|---|---|
| Munich | 19 December 2023 | Horváth, László |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................................................

& : member of the same patent family, corresponding
    document

EPO FORM 1503 03.82 (P04C01)

**EP 4 491 100 A1**

## ANNEX TO THE EUROPEAN SEARCH REPORT
## ON EUROPEAN PATENT APPLICATION NO.

EP 23 38 2718

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2023

| Patent document cited in search report | Publication date | Patent family member(s) | Publication date |
|---|---|---|---|
| CN 203520305 U | 02-04-2014 | NONE | |
| US 2017332901 A1 | 23-11-2017 | US 2017332901 A1 | 23-11-2017 |
| | | US 2018103843 A1 | 19-04-2018 |

EPO FORM P0459

For more details about this annex : see Official Journal of the European Patent Office, No. 12/82